# EUROPEAN PATENT APPLICATION

(11) **EP 0 700 991 A1**
(43) Date of publication of application: **13.03.1996**
(21) Application number: 95113609.2
(22) Date of filing: 30.08.1995
(51) Int. Cl.: C12N 1/10, A61K 39/012, G01N 33/569

(54) **Commercial scale concentrated preharvest tissue culture produced live T. Gondii bradyzoite and products and processes**

(30) Priority: 12.09.1994 US 304308
(71) Applicant: Bayer Corporation, Pittsburgh, PA 15219-2502 (US)
(72) Inventor: Brown, Karen K., Parkville, MO 64152 (US)
(74) Representative: Linkenheil, Dieter

(57) **Abstract**

Disclosed herein is a process for preparing pre-harvest tissue culture produced T. gondii that is suitable for a commercial scale vaccine for protection of cats against T. gondii; the vaccine contains an effective amount of bradyzoites and is free of or substantially free of tachyzoites.

## Description

### BACKGROUND OF THE INVENTION

### Field of the Invention:

The present invention relates to protection of cats against toxoplasmosis. More specifically, the invention relates to commercial scale concentrated tissue culture-produced live bradyzoites of Toxoplasma gondii (T. gondii), their vaccines and diagnostics and methods of making and using the same.

### Brief Description of the Prior Art:

Toxoplasmosis is an important parasitic disease of humans and domestic animals. The prior art reports it as a serious problem for seronegative pregnant women. If infected during pregnancy, the women may bear malformed or dead fetuses. The art has also reported it as a serious problem for immunocompromised individuals such as acquired immune deficiency syndrome (AIDS) patients and cancer patients on certain immunosuppressive drug therapies. Many of these individuals reportedly die of toxoplasmosis.

Cats are the definitive hosts in the complex life cycle of T. gondii. They become infected by ingesting small mammals, particularly mice which are infected by T. gondii and carry their cysts. A sexual replicative cycle of T. gondii occurs within the cat's gut, and results in the production of oocysts that are shed in the feces. The oocysts can infect other mammals via the oral route. Of particular concern here is the infection of humans either by contact with oocysts in soil and litter boxes or by eating undercooked meat of infected animals.

To protect against the infection, it would be desirable to have safe, commercial scale vaccines which would protect cats from infection and subsequent shedding of oocysts. Presently, the art-known commercial scale vaccines are conventionally prepared by growing the T. gondii in mouse brain. Heretofore, the art has not disclosed conventional methods for commercial-scale tissue culture production of T. gondii bradyzoites.

Illustrative of the prior art is Lindsay, et al (J. Parasitology, 1991 77(I), pages 126-132) which discloses tissue cyst formation by a goat isolate of T. gondii in: bovine monocytes, human fetal lung cells and Madin-Darby bovine kidney cells. However, cats fed with the resulting product shed oocysts in their feces. The disclosure teaches that one can use the preparation containing tissue culture growth of T. gondii to test drugs for activity against this organism. It does not teach the use of tissue culture to develop a vaccine. It does not teach the use of antibodies to produce bradyzoites in tissue culture preparations.

Jones et al (Infection and Immunity, Jan. 1986, pages 147-156) discloses an in vitro cultivation of T. gondii cysts in mouse astrocyte cells. It discloses an attempt to use gamma interferon to inhibit overgrowth of tissue culture cells with tachyzoites to allow the development of tissue cysts with bradyzoites. It does not teach the use of antibodies or enzymes. It does not teach the use of tissue culture-grown tissue cysts for production of a vaccine. It does teach that one would require 40-80 days to form significant numbers of tissue cysts. The cost of such long term growth would be prohibitive in commercial production of vaccines.

Shimada et al (Arch Ophthalmol., Vol 92, Dec 1974) discloses that one may grow the RH strain of T. gondii in rabbit corneal endothelial cells in Leighton tubes. It teaches the use antibody and guinea pig complement to attempt to inhibit growth of tachyzoites. It teaches that immune serum alone or guinea pig complement alone only minimally inhibit growth of tachyzoites, and that the combination of the two is synergistic. However, it does not teach the use of enzymes to kill tachyzoites or preparation or testing of a vaccine.

US Patent 4,824,666 discloses a mehtod of producing large scale growth of parasites of the genus Toxoplasma comprising: inoculating the tachyzoites of the microorganism into a non-adherent human cell line, such as monocytoid lymphoma cell line, amd maintaining the cell line in tissue culture media capable of supporting growth of the tachyzoites while the line is being maintained. The patent does not disclose large scale production of bradyzoites.

From the foregoing, it would be realized that the prior art has failed to meet the need of commercial scale concentrated tissue culture produced T. gondii vaccines. This failure may be due to the following associated problems. Perhaps the art perceived a problem of loss of immunogenicity in growing T. gondii in tissue culture. Perhaps the art perceived problems of antigen quantitation in T. gondii vaccines. Perhaps the art perceived the problem of maintaining the viability of the T. gondii bradyzoites in amounts that are sufficient to produce effective vaccines.

By the present invention, there is provided a safe, commercial scale tissue culture produced T. gondii and their vaccines and diagnostics.

### SUMMARY OF THE INVENTION

In accordance with the foregoing, the present invention encompasses pre-harvest, concentrated, commercial scale tissue culture-produced live T. gondii bradyzoites. The T. gondii useful herein can be of a strain that is characterized by an oocyst negative phenotype such as the strain that is designated as T-263.

The invention further encompasses a process for preparing pre-harvest, concentrated commercial scale tissue culture produced live T. gondii bradyzoites. In the present embodiment, the process comprises: (i) infecting a tissue culture with T. gondii tachyzoites; (ii) propagating T. gondii tachyzoites to a high yield; (iii) inhibiting the overgrowth of the tissue culture with the tachyzoites and stimulating the formation of bradyzoites in tissue cysts; (iv) propagating the resulting bradyzoites to a high yield; v) reducing the numbers of residual tachyzoites; (vi) releasing the bradyzoites from the cysts, and (vii) harvesting the tissue culture. In accordance with the invention, the tissue culture harvest precludes post-harvest concentration to formulate vaccines or or diagnostics.

Further, the invention encompasses vaccines and diagnostics containing effective amounts of the pre-harvest concentrated commercial scale tissue culture-produced live T. gondii bradyzoites. The vaccines contain immunogenically effective amounts of live T. gondii bradyzoites. The diagnostics contain effective amounts of T. gondii bradyzoites to provide a detectable immune complex. Also, the invention encompasses methods of administering the vaccines or diagnostics.

To aid in describing the invention more fully, some of the terms used herein are put in context as follows. The term "pre-harvest concentrated" denotes that the tissue culture produced live T. gondii bradyzoites are concentrated before harvesting. The term "commercial scale" denotes the absence of post-harvest concentration techniques such as centrifucation or ultrafiltration. Without using damaging, post harvest concentration techniques, one can obtain immunogenically effective amounts of live bradyzoites in consistently high yields that are antigenically quantifiable. The term "immunogenically effective amount" means the amount of live bradyzoites that can be formulated into a vaccine to protect cats from shedding of oocysts.

The term "antigenically quantifiable" means the ability to accurately measure the number of live bradyzoites in a dose of vaccine that confers protection. One can determine the antigenically quantifiable amounts of the T. gondii by assay methods which involve either an injection of mice followed by a detection of T. gondii antibody, or an inoculation of tissue culture followed by an observation of cytopathic effect (CPE). As would be realized, accurate quantitation of the antigenic materials would be required for commercial or regulatory purposes.

It has been found that the pre-harvest concentrated tissue culture produced live T. gondii bradyzoites can be safely prepared on a commercial scale. It has also been found that their vaccines are particularly useful in protecting against toxoplasmosis. The invention is described more fully hereunder.

### DETAILED DESCRIPTION OF THE INVENTION

As set forth above, the present invention comprises pre-harvest concentrated commercial scale tissue culture-produced live T. gondii bradyzoites. Generally, the T. gondii useful herein is that which contains a protective antigen and lacks reproductive capability. By the term "lacks reproductive capability" is meant the T. gondii strain cannot produce oocysts which could be shed to the environment. An illustrative but non-limiting example of the T. gondii useful herein can be a T-263 mutant. This mutant has been deposited with the American Type Culture Collection and has been accorded ATCC Accession No. 40615.

Modified live or attenuated mutants may be produced by exposing the tachyzoites of any T. gondii strain such as the "C" strain to an alkylating agent such as N-methyl-N-nitro-nitroso-guanidine [Pffefferkron, E.R. and Pfefferkron, L.C., "Toxoplasma gondii: Isolation and Preliminary Characterization of Temperature Sensitive Mutants." Experimental Parasitology 39, 365-376, (1976)], and isolating mutants which are resistant to one or more antimetabolites. The resistant mutants are then selected further for the oocyst negative phenotype and, finally selected for their ability to protect cats.

Any tissue culture primary cell or cell line which supports the growth of T. gondii may be used according to this invention. Illustratively, the tissue culture cell or cell line can be selected from the group consisting of: embryonic bovine lung cells, mouse astrocyte cells, a Human Foreskin Fibroblast cell line and a Human Diploid Lung cell (MRC-5). Also, the Crandell cell line (feline), Cutter Laboratories Dog Kidney Cell Line (CLDK), Vero Cell Line and Madin-Darby Bovine Kidney (MDBK) cell lines can be used. In particular, slow-growing cells such as the embryonic bovine lung cell, are useful in growing live T. gondii bradyzoites. These cells are particularly suitable to the specific growth conditions of the T. gondii of the invention.

As would be realized, the afore-mentioned process steps of (i) infecting a tissue culture with T. gondii tachyzoites and (ii) propagating the T. gondii tachyzoites can be considered as a step of providing a high yield of tachyzoites in a tissue culture. The resulting tissue culture containing T. gondii tachyzoites can be subjected to steps (iii) through (vii) that are also mentioned above. In a specific but non-limiting embodiment, the process comprises growing a tissue culture to a high cell count by art-known techniques. While the techniques for growing the tissue culture to a high cell count is art-known, the recognition that a high cell count is required here is considered as a part of the invention. The tissue culture having a high cell count is infected with T. gondii tachyzoites, at a multiplicity of infection (MOI) to produce the high yield of tachyzoites. The term "multiplicity of infection (MOI)" means the ratio of the number of T. gondii tachyzoites used in infecting the cell culture to the actual count of cells (number) which produces the highest number of organisms infecting each cell. Useful herein is an MOI of from 0.001 to 1.0, and preferably 0.01 to 0.5. Typically, the MOI is 0.05. The T. gondii tachyzoites are propagated under conducive tissue culture growth conditions to produce a high yield of tachyzoites. The conditions comprise a select tachyzoite:cell:growth medium ratio that is effective to produce the highest count (number) of tachyzoites per mililiter of growth medium. The growth medium required to support the maximum growth of cells (maximum cell count/mL) can be selected from the group consisting of Dulbecco's Minimum Essential Medium (DMEM), Earl's Lactalbumin Hydrolysate, Earl's Minimum Essential Medium, high glucuose, DMEM with glucose and glutamine. The growth medium required to support the maximum growth (high yied) of tachyzoites comprises high glucose, DMEM with glutamine and fetal bovine serum. The amount of medium could vary from 200 mL to 1000 mL per 1750 cm² surface. Preferably 300 to 500 mL of media would be used. The tissue culture growth conditions futher comprise a pH between 6.5 and 7.5 and a temperature between 34 and 39°C. Preferably, the conditions comprise a pH between 6.8 and 7.2 and a temperature between 35 and 37°C. The resulting tachyzoite count should be at least 1 X 10⁵/mL of tissue culture fluid.

When the tachyzoites have grown to sufficiently high levels such that cytopathic effect is observed, the growth of the tachyzoites (aptly referred to herein as overgrowth) is inhibited and tachyzoites are stimulated to form tissue cysts. This can be done by addition of T. gondii -specific antibody and preferably T. gondii tachyzoite specific antibody to the tissue culture. The tachyzoite-specific antibody is added in an amount sufficient to inhibit the overgrowth of the tissue culture by the tachyzoites. Concentrations of 0.1% to 10% and preferably 3% to 5% can be employed. Illustrative but non-limiting examples of the tachyzoite-specific antibody can be selected from the group consisting of goat, rabbit, horse and bovine. Resulting from the foregoing are bradyzoites in the tissue cysts.

The bradyzoites in the tissue cysts are propagated to such a high yield that an appreciable number of them would survive an attrition that is due to conditions of storage and/or vaccine formulation. The term "high yield" means a large number of pre-harvest, tissue culture produced, live T. gondii bradyzoites that can be formulated into an effective vaccine without concentrating the harvested tissue culture. The high yields of live bradyzoites are required here because of the high rates of attrition of live bradyzoites under the storage and formulation conditions. Illustratively, vaccine formulation steps such as an addition of stabilizing chemicals, freezing in liquid nitrogen and lyophilization can result in a 2 to 4 log reduction in bradyzoite viability. It is, therefore, advantageous to provide a high yield of the live bradyzoites, e.g., about 2.5 X 10⁵ live bradyzoites in each mililiter of the tissue culture harvest.

The number of residual tachyzoites in the tissue culture is reduced and the live bradyzoites are released from the tissue cysts by addition of enzymes to the tissue culture.The proteolytic enzyme can be employed in a concentration from 0.0001% to 5% and preferably from 0.0001% to 0.1% w/v. The enzyme useful herein can be a proteolytic enzyme that is selected from the group consisting of pepsin, trypsin and chymotrypsin.

The resulting tissue culture material can be harvested by chemical or mechanical removal of the cells, tissue cysts and live bradyzoites which may be attached to the vessel surface. Mechanical means can comprise the use of glass beads, rapid agitation, or scraping. Chemical means can comprise an addition of enzymes such as trypsin. In preparing a pre-harvest concentrated tissue culture of T. gondii bradyzoites in commercial scale one typically employs large volume tissue culture systems (vessels) such as: roller bottles, bioreactors or the like vessels. Bioreactors are preferred. In the use of these vessels, at least 10 liters of bradyzoite harvest material can be obtained per each batch. Illustratively, the commercial scale tissue culture described in this invention can produce as many as 500,000 bradyzoites per mililiter of harvest fluid. Therefore, the present invention provides a facile and cost effective process for preparing live T. gondii bradyzoites. In contrast, a commercial scale T. gondii preparation in mice produces about 10 doses of vaccine per mouse. The mice must be injected with T. gondii using a syringe/needle (an unsafe practice), held and fed and cared for at least 28 days and then harvested. The process is extremely labor-intensive and costly.

It is also a feature of the invention that the process of the invention is typically conducted in closed systems (vessels). Hence, no syringes and needles are required for inoculation of cells. In effect, the use of the closed systems prevents the infection of individuals conducting the process. The present invention therefore provides a safe process for preparing the tissue culture produced live bradyzoites and vaccines.

The vaccines or diagnostics can be formulated with the live bradyzoites of the invention. The vaccines can be in a liquid, frozen or lyophilized state and can be formulated by stabilizing the harvested tissue culture by addition of chemicals such as dimethylsulfoxide (DMSO), sugars and serums. Typically, the preserved material is frozen in liquid nitrogen. As low as 10 bradyzoites per dose can provide protection against oocyst shedding. Typically the vaccines contain from about 100 to 200 bradyzoites per dose. It is a distinct feature of the invention that the vaccines consist essentially of bradyzoites. That is the vaccines are free or substantially free of tachyzoites.

As would be realized, the presence of tachyzoites with the bradyzoites in vaccines could cause a false antigen quantitation because tachyzoites do not protect cats against oocyst sheding. A vaccine containing tachyzoites as well as bradyzoites could produce false-positive seroconversion in diagnostic tests of cats. In effect, the presence of a significant number of tachyzoites in a T. gondii vaccine would "interfere" with the effectiveness of a commercial scale tissue culture produced T.gondii vaccine. To allow a reliable antigen quantitation of vaccines containing tissue culture produced T. gondii, one has to control the tachyzoite content of the vaccines. The requirement of essentially precluding tachyzoites from the vaccine is even more significant, since tachyzoites grow better than bradyzoites in tissue culture. From the regulatory and/or commercial viewpoint, the presence of a substantial number of tachyzoites in a vaccine would be undesirable.

In the practice of the invention the vaccine can be administered to cats to protect them against oocyst shedding. The administration is made orally via a unique straw technology described in co-pending U.S. Patent Application Serial Number08/118,905 that is incorporated herein by reference. Other methods of administration to cats would include intramuscular, subcutaneous, intra-rectal or intra-vaginal.

Also in the practice of the invention, diagnostics containing the tissue culture produced T. gondii can be used in detecting the presence or absence of toxoplasmosis. Currently, all diagnostic tests for detection of T. gondii infection contain tachyzoites. Live bradyzoites that are produced according to this invention would be better suited to use as an antigen in diagnostic tests. This is because live bradyzoites are protective for cats whereas live tachyzoites, although able to stimulate an antibody response in cats, cannot protect them. Live or inactivated (killed) bradyzoites that are produced according to this invention may be used to coat a matrix that is useful in diagnostic test systems. Examples thereof can be: ELISA plates, polymer or filter strips or the like. After coating of the matrix with live or killed bradyzoites, the matrix may be mixed with reagents and serum from animals. If the serum is from an animal which has had a T. gondii infection and is protected from reexposure, the use of bradyzoites (instead of tachyzoites )should detect this protective state.

The following examples further illustrate specific embodiments of the invention. The examples, however, are not meant to limit the scope of the invention which has been fully characterized in the foregoing disclosure.

### EXAMPLE 1

A specific example of tissue culture growth of T-263 tissue cysts containing bradyzoites is described as follows. A vial of working seed tachyzoites originating from Master Seed produced in mouse peritoneal exudates and containing 5 X 10⁵/mL tachyzoites was thawed and added to a confluent Human Foreskin Fibroblast (HSF) monolayer culture containing 1 X 10⁶ cells. The MOI was thus 0.5. The tissue culture were allowed to progress until the cytopathic effect (CPE) was complete. This first tissue culture growth (passage) was conducted in order to adapt tachyzoites to tissue culture. Embryonic Bovine Lung (EBL) cells were grown in 75cm2 flasks using Dulbeccos Minimal Essential Medium DMEM + 10% Fetal Bovine Serum (FBS) + glutamine and gentamicin. When the cells were confluent, the medium was removed and 12 ml/flask of Toxoplasma Maintenance Medium (TMM = DMEM with 4500 mg/mL glucose, 3% FBS, 1% glutamine at 2mM and 50mg/mL gentamicin) was added. This was followed by addition of 5 x 10⁵ tachyzoites from the HSF cell culture. The flasks were incubated at 37°C in an atmosphere of 5% CO2 for 4 days. On day 4, fresh TMM plus 5% goat or rabbit anti-toxoplasma serum determined to be positive by ELISA was added to each flask. Flasks were incubated 3 more days under the previously-described conditions. On day 7, the cysts were harvested by scraping the cells off the growing surface. Using this method, there were obtained approximately 200 cysts with approximately 100,000 bradyzoites per flask or approximately 8,000 live bradyzoites per mililiter to tissue culture harvest.

These live tissue cysts containing bradyzoites were used to prepare a vaccine. Vaccines were prepared in which the tissue cysts were left intact (NON PEPSIN TREATED) or were digested tor l0 min. at 37°C with 0.05% pepsin (PEPSIN DIGESTED). The latter treatment not only killed the remaining tachyzoites but also released the bradyzoites. The vaccine formulations were diluted to contain specific amounts of tissue cysts or bradyzoites and each formulation was back titrated in mice at the time of cat vaccination.

Five cats were vaccinated with either 10, 100 or 1000 bradyzoites which were in the form of NON PEPSIN TREATED tissue cysts. An additional 5 cats (per formulation) were vaccinated with either 10, 100 or 1000 bradyzoites which were in the form of PEPSIN DIGESTED cysts. The bradyzoite concentrations per dose were confirmed by back titration in mice. All cats were boostered with formulations containing 1, 10 or 100 bradyzoites respectively at 21 days post primary vaccination. On day 43 post primary vaccination all cats were challenged with 10,000 bradyzoites of a virulent oocyst-shedding T. gondii strain designated T-265. Five cats which were not vaccinated were challenged to serve as controls. Fecal samples were examined for oocyst shedding on days three to fourteen post vaccination and booster and for 14 days post challenge. Such fecal examination involved a microscopic evaluation of fecal samples for oocysts. In addition, the fecal samples were centrifuged and supernatant was removed and injected IP into mice. Four weeks after injection, the mice were bled and checked for seroconversion to T. gondii. Seroconversion of mice injected with fecal supernatants (indirect agglutination titer is ≧1:40) indicates that there was at least one oocyst shed in the fecal sample tested.

Table I shows that no oocysts were shed post vaccination or booster with any of the formulations. It is noted that five of the six vaccines produced protection in cats. One hundred percent (5/5) of control cats were infected and shed oocysts post challenge. Analysis of the results of the individual vaccines indicates that the PEPSIN TREATED cyst vaccine produced more consistent protection. The dose containing 10 bradyzoites protected 40% of the cats post challenge. The vaccine containing 100 bradyzoites protected 80% of the cats. The vaccine containing 1000 bradyzoites protected 60% of the cats post challenge. Vaccines prepared from cysts which were NON PEPSIN TREATED, (bradyzoites were not released from the cysts and there was contamination with tachyzoites) protected: 80% of the cats at a dose level of 10 bradyzoites, 0% at a dose level of 100 bradyzoites and 75% at a dose level of 1000 bradyzoites. This inconsistency is not desirable for a commercial product. Therefore, PEPSIN DIGESTION of tissue culture grown tissue cysts which results in release of the bradyzoites and destruction of the tachyzoites is preferable for commercial production of a vaccine.

The vaccine can be administered to cats in any desirable manner. Presently, an oral mode of administration is preferred. Dosages of 0.1 mL to 2.0 mL and preferably 0.2mL to 1.0 mL per cat can be employed. While the vaccines described hereinabove are liquid, it is within the purview of the skilled artisan to make and use lyophilized or frozen forms of the vaccine.

It has been demonstrated that tissue culture-grown T. gondii vaccine can immunize cats and thus protect them from shedding when they are later exposed to a virulent shedding strain. Additionally, bradyzoites prepared in tissue culture can be used for development of diagnostic assays. Table 1, further illustrates the invention.

### EXAMPLE 2

The following further illustrates the growth conditions for preparing a pre-harvest concentrated tissue culture of T. gondii bradyzoites in a roller bottle.

Preparation of Working Seed. Rapidly growing cells such as HSF cells are used to produce a high yield of working seed T. gondii tachyzoites from the Master Seed via procedures described in Example 1.

Maximization of EBL cell growth in the minimal volume of medium. EBL cells are planted into roller bottles containing different volumes of cell growth medium. The media used is DMEM + 10% Fetal Bovine Serum. Cell counts are made on samples from these roller bottles at 8 hour intervals in order to determine the maximum growth rate in the least amount of media. The maximum time for infection with the Working Seed is determined by evaluating the time it takes for production of the maximum number of healthy cells in the least volume of medium. It has been found that 300 mL of Dulbecco's Minimal Essential Medium (DMEM) + 10% Fetal Bovine Serum will grow these cells to confluency within 7 days in 9L roller bottles.

Determination of MOI. When the EBL cells from the previous step are confluent (1 X 10⁶/mL), they are refed with fresh media containing DMEM + 4.5 g/mL glucose, 3% FBS, 1% glutamine at 2mM and 50 mg/mL gentamicin. They are then infected with the HSF-grown tachyzoites at counts ranging from 1 X 10³/mL to 1 X 10⁷/mL of tissue culture media. Samples are taken during propagation of the tachyzoites and tachyzoite numbers in the 1 mL samples are counted. The MOI which produces the most tachyzoites per mililiter of tissue culture media is calculated. Such MOI is generally between 0.01 and 0.5 with the best MOI for the roller bottle system being 0.05. The MOI provides the number of T. gondii tachyzoites required to infect each cell so as to produce the maximum yield of T. gondii tachyzoites per mililiter of tissue culture medium.

Maximizing growth of tachyzoites. After the MOI is determined, a new set of roller bottles is prepared. These roller bottles contain the media defined in the previous steps with various buffers added to control the pH of the tissue culture at ranges between pH 6.0 and 8.0. Incubation temperature (37°C), oxygen tension and CO₂ levels are held constant for this experiment. Samples are removed at 8-12 hour intervals and tachyzoites are counted. The pH at which the maximum number of tachyzoites are produced is between 6.8 and 7.4. The maximum yield of tachyzoites is produced at temperatures between 35 and 37°C. Therefore, tachyzoites are maximally produced at a pH between 6.8 and 7.4, a temperature between around 37°C with no additional oxygen or CO₂ provided.

Propagation of high yield bradyzoites. Tachyzoites are stimulated to convert to their next stage of development (tissue cysts containing bradyzoites) when their environment becomes deterimental to their survival. Propagation of bradyzoites begins with stimulating the tachyzoites in the tissue culture to convert to tissue cysts. This is accomplished by refeeding the infected tissue culture with fresh medium containing DMEM + 4.5 g/mL glucose, 3% FBS, 1% glutamine at 2mM, 50 mg/mL gentamicin and T. gondii tachyzoite-specific antisera. Such antisera is added in amounts between 0.1 and 10%, preferably around 5%. After addition of the antisera, the conditions for growth of tachyzoites are minimized and they begin to transform to tissue cysts which contain bradyzoites. At the point where tissue cysts begin to form, there is provided a growth medium (fresh medium which is the same as that described previously in this section) which contains fresh nutrients specific for propogation of bradyzoites. The tissue culture incubation is continued until a maximum bradyzoite count of at least 2.5 X 10⁵/mL of tissue culture fluid occurs. The count is determined by: microscopic observation of the culture, followed by counting of bradyzoites in the cysts and, additionally, counting the number of cysts in a specified area of the tissue culture surface (cysts per cm² in a roller bottle or cysts per bead in a bioreactor).

Removal of residual tachyzoites. When the bradyzoite count is at maximum, the tissue culture is treated with an enzyme such as pepsin, trypsin or chymotrypsin at a concentration ranging between 0.01 and 1.0%.

Harvest of the bradyzoites. After the enzyme treatment step, the tissue culture is harvested. Harvest can be conducted by asceptically removing cells, tissue cysts and bradyzoites, by adding further enzymes to the tissue culture (trypsin at concentrations up to 1.0%) or by mechanically removing the cells such as by adding glass beads or similar matrices to scrape the cells off of the surface to which they are attached. When harvest is complete the tissue culture harvest material should contain at least 2.5 X 10⁵ bradyzoites/mL and these can be formulated directly into vaccine without post-harvest concentration.

Formulation into a vaccine for cats. The tissue culture harvest is stabilized by addition of chemicals such as dimethyl sulfoxide (DMSO) and FBS, filled into a straw delivery system at 0.2 mL/straw and frozen in liquid nitrogen. Such a process adversely affects the live bradyzoites such that from 2 to 4 logs of viability of the bradyzoites are lost. The live bradyzoites may also be frozen and lyophilized with similar losses in viability being noted.

Administration of T. gondii live bradyzoite vaccine. Frozen straws containing the live bradyzoites are removed from the liquid nitrogen, thawed and delivered orally to cats in order to immunize them. The immunization schedule and testing procedure are described in Example 1.

### EXAMPLE 3

Further illustrating the invention are the following. Where roller bottles are used for pre-harvest concentrated, commercial scale production, the amount of medium could vary from 200 mL to 1000 mL per 1750 cm² surface. The pH should be between 6.6 and 7.5 Temperature control should remain between 35 and 39°C. EBL cells are grown in DMEM + 10% FBS until they reach 1 X 10⁶ cells/mL. Cells are infected with tachyzoites at an MOI of 0.01 to 0.05 in a fresh medium containing DMEM, 4.5 g/mL glucose, 3% FBS, 1% 2mM glutamine, and 50 mg/mL gentamicin. Tachyzoites are allowed to grow to a maximal count over a period of from 3 to 5 days at 37°C. At this time, fresh medium (defined above) + T. gondii tachyzoite-specific antibody at 3-5% is added to the infected tissue culture. The roller bottles are incubated for 3-4 more days during which the tissue cultures are microscopically observed for development of tissue cysts containing bradyzoites. When the maximum count of bradyzoites is reached, 0.05% pepsin and 5 g of glass beads are added to each roller bottle and allowed to incubate for an additional 30 to 60 minutes. The contents of the roller bottles are pooled by transferring them through a collecting device which separates out the glass beads. This harvested tissue culture containing bradyzoites at >2.5 X 10⁶ can then be used for formulation of a vaccine or preparation of a diagnostic reagent. When T. gondii is grown on a commercial scale in tissue culture, one roller bottle containing from 200 mL to 1L of tissue culture media can produce as many as 500 doses of vaccine. The time required for growth is approximately 7 to 10 days.

Bradyzoite antigen prepared in large-scale tissue culture according to this invention can protect cats from shedding post challenge with a wild-type T. gondii, i.e., prevent oocyst shedding of a heterologous strain, i.e., T-265. The tables hereinbelow show the tissue culture vaccination study that is based on Example 1.

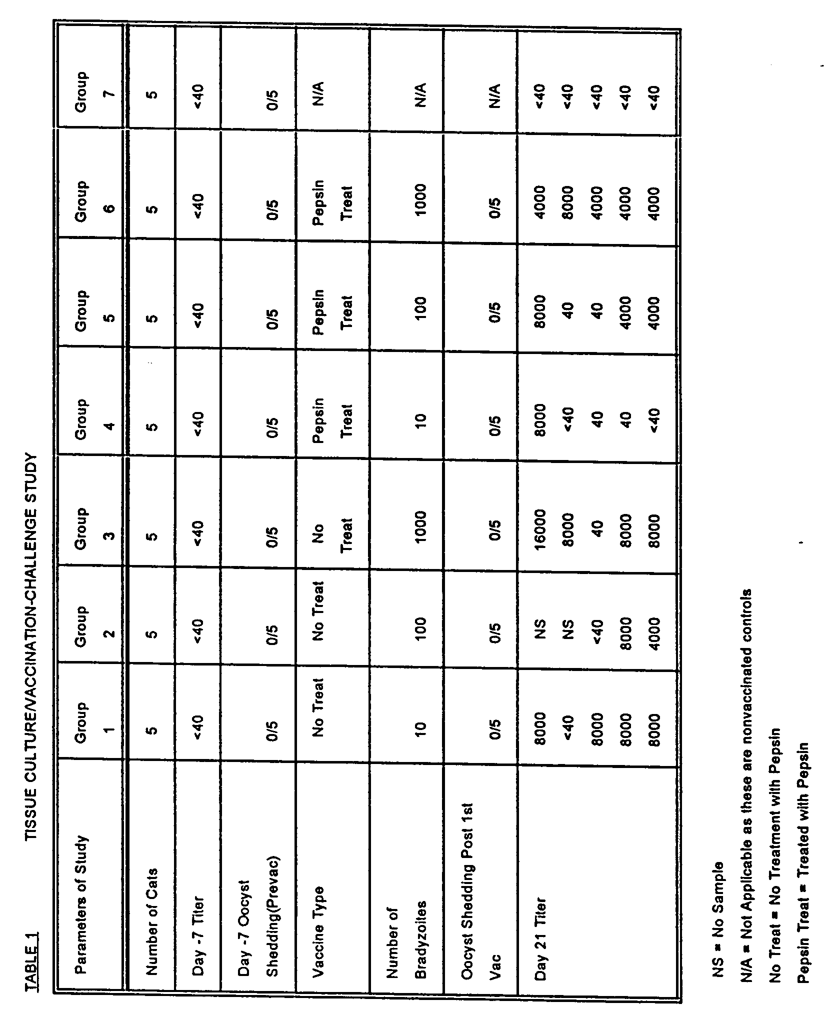

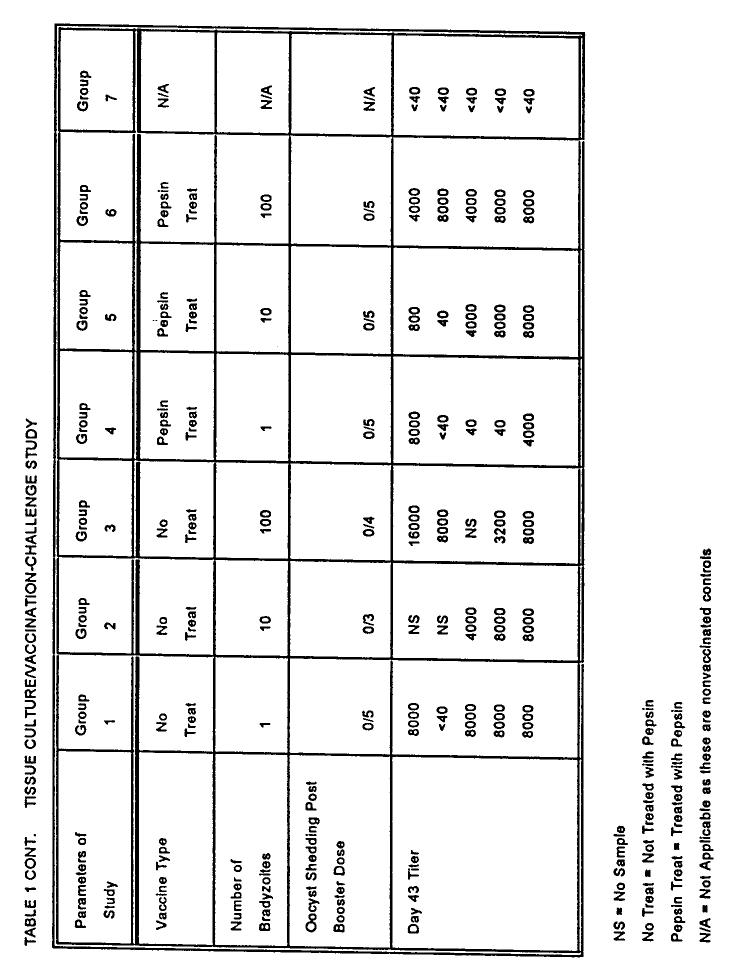

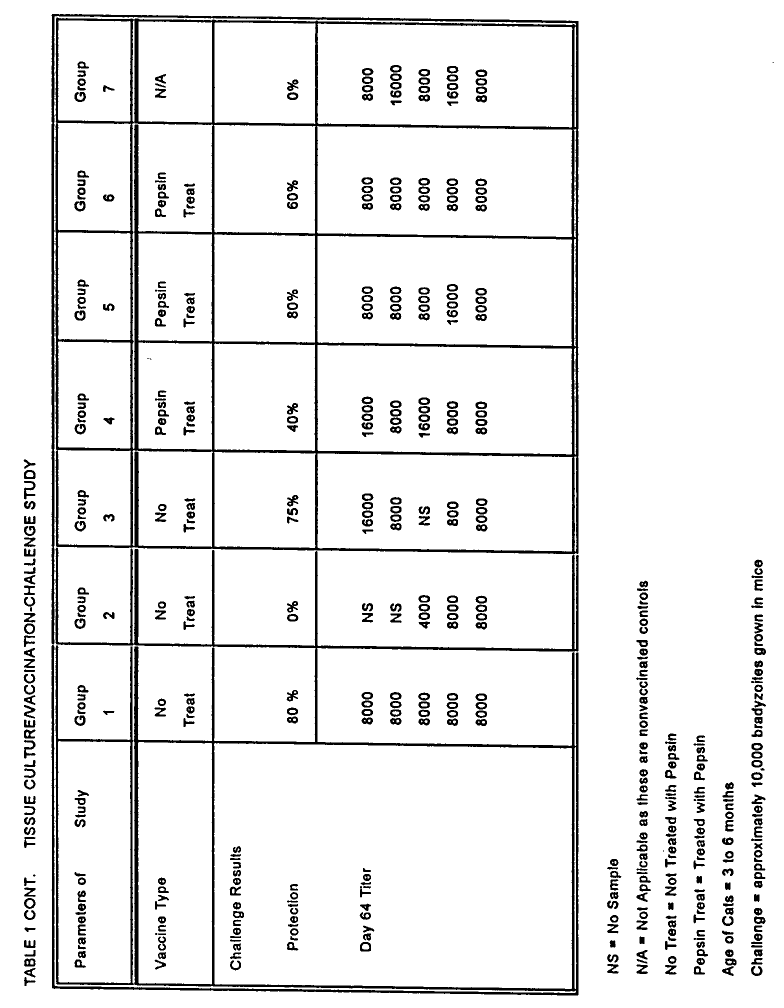

Although the invention has been described in detail in the foregoing for purpose of illustration, it is to be understood that such detail is solely for that purpose and that variations can be made therein by those skilled in the art without departing from the spirit and scope of the invention except as it may be limited by the claims.

## Claims

1. Pre-harvest concentrated commercial scale tissue culture-produced live T. gondii bradyzoites.

2. The T. gondii as recited in Claim 1 which is of a strain that is characterized by an oocyst negative phenotype.

3. The T. gondii as recited in Claim 1 which is of a strain that is designated T-263.

4. A process for preparing pre-harvest concentrated commercial scale tissue culture-produced live T. gondii bradyzoites comprising:
i) infecting a tissue culture with T. gondii tachyzoites, ii) propagating T. gondii tachyzoites to a high yield, iii) inhibiting the overgrowth of the tissue culture with the tachyzoites and stimulating the formation of bradyzoites in tissue cysts, iv) propagating the resulting bradyzoites to a high yield, v) reducing the numbers of residual tachyzoites vi) releasing the bradyzoites from the cysts, and vii) harvesting the tissue culture.

5. The process of Claim 4 wherein the tissue culture is derived from a member selected from the group consisting of embryonic bovine lung cells, mouse astrocyte cells, human foreskin fibroblast cells and MRC-5 cells

6. The process of Claim 4 wherein the tissue culture is infected at a multiplicity of infection that is effective to produce a high yield of tachyzoites.

7. The process of Claim 4 wherein the tachyzoites overgrowth is inhibited by an addition of T. gondii-specific antibody to the tissue culture to produce bradyzoites

8. The process of Claim 7 wherein the T. gondii-specific antibody is is a member selected from the group consisting of goat, rabbit, horse and bovine.

9. The process of Claim 4 wherein the reduction of the number of residual tachyzoites is by adding a proteolytic enzyme selected from the group consisting of pepsin, trypsin and chymotrypsin.

10. A vaccine containing an immunogenically effective amount of pre-harvest concentrated tissue culture-produced live T. gondii bradyzoites as recited in Claim 1.

11. The vaccine of Claim 10 characterized in that it is live or modified live bradyzoites.

12. The vaccine of Claim 10 which is in a liquid, frozen or lyophilized state.

13. A diagnostic for detection of toxoplasmosis containing the pre-harvest concentrated live T. gondii bradyzoites of Claim 1.

14. A method of treating a cat to prevent it from shedding T. gondii oocysts comprising administering the vaccine of Claim 10 to the cat.

15. A method of diagnosing Toxoplasmosis in a cat comprising contacting a bioligical sample with the diagnostic material of Claim 13 and detecting the presence or absence of an immune complex.
